(19) Europäisches Patentamt

European Patent Office

Office européen des brevets

(11) **EP 1 015 414 B1**

(12) **EUROPÄISCHE PATENTSCHRIFT**

(45) Veröffentlichungstag und Bekanntmachung des Hinweises auf die Patenterteilung:
**20.04.2005 Patentblatt 2005/16**

(21) Anmeldenummer: **98946431.8**

(22) Anmeldetag: **26.08.1998**

(51) Int Cl.[7]: **C07C 205/06**, C07C 201/08

(86) Internationale Anmeldenummer:
**PCT/EP1998/005408**

(87) Internationale Veröffentlichungsnummer:
**WO 1999/012887 (18.03.1999 Gazette 1999/11)**

(54) **VERFAHREN ZUR HERSTELLUNG EINES DINITRONAPHTHALIN-ISOMERENGEMISCHES MIT HOHEM ANTEIL AN 1,5-DINITRONAPHTHALIN**

METHOD FOR PRODUCING A DINITRONAPHTHALENE-ISOMER MIXTURE HAVING A HIGH 1,5-DINITRONAPHTHALENE PROPORTION

PROCEDE DE PRODUCTION D'UN MELANGE D'ISOMERES DE DINITRONAPHTALENE A PROPORTION ELEVEE EN 1,5-DINITRONAPHTALENE

(84) Benannte Vertragsstaaten:
**BE DE ES FR GB IT NL**

(30) Priorität: **08.09.1997 DE 19739202**

(43) Veröffentlichungstag der Anmeldung:
**05.07.2000 Patentblatt 2000/27**

(73) Patentinhaber: **Bayer MaterialScience AG
51368 Leverkusen (DE)**

(72) Erfinder:
• **STEINLEIN, Christian
D-41469 Neuss (DE)**
• **WEGENER, Gerhard
D-40822 Mettmann (DE)**

(56) Entgegenhaltungen:
**FR-A- 1 248 426      US-A- 3 976 704**

• **CHEMICAL ABSTRACTS, vol. 99, no. 5, 1. August 1983 Columbus, Ohio, US; abstract no. 38110t, KAMEO TAKASHI ET AL.: "Studies on nitration of aromatic comounds." Seite 493; Spalte 1; XP002088928 & NIPPON KAGAKU KAISHI, Bd. 3, 1983, Seiten 414-419,**
• **GEORGE A. OLAH ET AL.: "Aromatic substitution." JOURNAL OF ORGANIC CHEMISTRY., Bd. 47, 1982, Seiten 596-598, XP002088925 EASTON US**
• **GEORGE A. OLAH ET AL.: "Aromatic substitution." JOURNAL OF ORGANIC CHEMISTRY., Bd. 43, Nr. 24, 1978, Seiten 4628-4630, XP002088926 EASTON US**
• **CHEMICAL ABSTRACTS, vol. 124, no. 10, 4. März 1996 Columbus, Ohio, US; abstract no. 120703g, JING, YINGJIE ET AL.: "Selective catalytic nitration of toluene" Seite 200; Spalte 2; XP002088929 & FUSHUN SHIYOU XUEYUAN XUEBAO, Bd. 15, Nr. 3, 1995, Seiten 12-15,**
• **MARK A. HARMER ET AL.: "High surface area nafion resin/silica nanocomposites: a new class of solid catalyst" JOURNAL OF THE AMERICAN CHEMICAL SOCIETY., Bd. 118, Nr. 33, 21. August 1996, Seiten 7708-7715, XP002088927 DC US**
• **1988, VCH, INDUSTRIELLE ORGANISCHE CHEMIE, D-WEINHEIM Seiten 395 - 396**

## Beschreibung

Die Erfindung betrifft die Herstellung eines Dinitronaphthalin-Isomerengemisches mit erhöhtem Anteil an 1,5-Dinitronaphthalin (nachfolgend 1,5-DNN genannt) durch die Nitrierung von reinem 1-Nitronaphthalin oder einem Nitronaphthalin-Rohgemisch. Das 1,5-Dinitronaphthalin stellt eine Schlüsselverbindung zur Herstellung des 1,5-Diaminonaphthalins (nachfolgend 1,5-NDA genannt) dar. Dieses ist u.a. die Ausgangsverbindung zur Herstellung des 1,5-Diisocyanatonaphthalins (1,5-NDI, Handelsnamen Desmodur 15®). Das 1,5-NDI wird als Isocyanatkomponente in Polyurethanen eingesetzt.

Die Herstellung von nitrierten Aromaten ist seit langem bekannt (G.A. Olah et al., Nitration: Methods and Mechanismus, VCH, New York, 1989). Seit Jahrzehnten werden entsprechende Nitroaromaten durch Nitrierung mit einer Mischung aus Schwefel- und Salpetersäure (sogenannte Misch- oder Nitriersäure) technisch hergestellt. Mehrfache Nitrierungen, beispielsweise Dinitrierungen, werden heute meist durch ein zweistufiges Nitrieren großtechnisch durchgeführt (Kirk-Othmer; Encyclopedia of Chemical Technology, 1981, Vol. 15 und Ullmann; Encyclopedia of Industrial Chemistry, 1991, Vol. A17, Seiten 411-455).

Die-Nitrierung von Naphthalin (Houben-Weyl; Methoden der Organischen Chemie, 1971, Vol. 10, Seiten 492-495) liefert ein Isomerengemisch von 1-Nitronaphthalin und 2-Nitronaphthalin im Verhältnis von etwa 95:5. Bei der Nitrierung des isomerenreinen 1-Nitronaphthalins mit einer Mischung aus Schwefel- und Salpetersäure entsteht eine Mischung von 1,5- und 1,8-Dinitronaphthalin im Verhältnis von etwa 1:2 neben etwa 5 % der anderen Isomeren (z.B. 1,6- und 1,7-DNN). Die ungünstige Selektivität der Reaktion führt bei der Herstellung des 1,5-DNN vorzugsweise zu einem hohen und unerwünschten Anteil an 1,8-DNN.

In der DE-A 11 50 965 wird die Erhöhung der Selektivität durch ein schnelles und intensives Vermischen des in Schwefelsäure gelösten 1-Nitronaphthalins mit Nitriersäure beschrieben. Nachteil dieses Verfahrens ist die erhebliche Menge an Schwefelsäure, die als Lösungsmittel eingesetzt wird und dessen Aufarbeitung sehr aufwendig und kostenintensiv ist. Außerdem können bei diesem Verfahren erhebliche Mengen an trinitrierten Produkten entstehen, die sowohl die 1,5-DNN-Ausbeute deutlich mindern als auch sicherheitstechnisch als kritisch anzusehen sind; insbesondere bei der im genannten Stand der Technik beschriebenen adiabatischen Reaktionsführung.

In der WO 94/19310 sind Nitrierungen von Nitroaromaten an mit teilweise Schwermetall-dotierten Aluminiumsilikaten, sogenannten Claycops, als festen Katalysator beschrieben, die hohe Ausbeuten an dinitrierten Produkten bei geringen Mengen an Trinitroaromaten liefern. Nach diesem Verfahren durchgeführte Nitrierungen von 1-Nitronaphthalin liefern allerdings Isomerenverhältnisse wie bei klassischen Nitrierungen mit Mischsäure.

FR-A-1248426 offenbart ein Verfahren zur Herstellung eines Dinitronaphthalin-Isomerengemisches durch Nitrierung von 1-Nitronaphthalin. In dem Verfahren gemäß FR-A-1248426 werden jedoch keine festen stark sauren perfluorierten Ionenaustauscher als Katalysatoren eingesetzt.

Die Schriften Chemical Abstract, Band 99, 1983, N. 38110t, J. Org. Chem., 1982, Band 47, 596 - 598 sowie US-A-3,976,704 offenbaren Verfahren zur Nitrierung von Aromaten in Gegenwart saurer perfluorierter Ionenaustauscher, wobei diese Nitrierverfahren jedoch ausschließlich die Erstnitrierung und nicht die Zweitnitrierung von Aromaten umfassen.

Nitrierungen mit Salpetersäure in organischen Lösungsmitteln, z.B. Dichlorethan, und azeotroper Entfernung des Reaktionswassers sind in der DE-A 24 53 529 beschrieben. Sie liefern Dinitronaphthalin in hohen Ausbeuten ohne jedoch das Isomerenverhältnis zu beeinflussen.

Es bestand daher die Aufgabe, ein Verfahren zur Herstellung eines Dinitronaphthalin-Isomerengemisches mit hohem Anteil an 1,5-Dinitronaphthalin durch Nitrierung von 1-Nitronaphthalin aufzufinden, bei dem keine nur aufwendig aufzuarbeitende Misch- oder Nitriersäure eingesetzt werden muß.

Überraschenderweise wurde nun gefunden, daß bei der Verwendung von festen perfluorierten, stark sauren Ionenaustauschern als Katalysator bei der Nitrierung von 1-Nitronaphthalin eine Verschiebung des Isomerenverhältnisses hin zum 1,5-DNN möglich ist. Die Reaktion wird in einem Überschuß Salpetersäure durchgeführt, welche nach erfolgter Reaktion und Aufkonzentrierung wiedergewonnen werden kann.

Gegenstand der Erfindung ist somit ein Verfahren zur Herstellung eines Dinitronaphthalin-Isomerengemisches durch Nitrierung von Nitronaphthalin, bei dem man Nitronaphthalin gegebenenfalls in einem inerten organischen Lösungsmittel mit der 1 bis 20-fachen Äquivalentmenge, bezogen auf Nitronaphthalin, an 50 bis 100 %iger Salpetersäure in Gegenwart eines festen perfluorierten stark sauren Ionenaustauschers umsetzt. Die 1-20-fache Äquivalentmenge entspricht bei einer z.B. 65 %igen Salpetersäure 33 Gew.-% bis 92 Gew.-% bezogen auf die Lösung, bei einer 100%igen Salpetersäure von 26 Gew.-% bis 88 Gew.-%.

Die erfindungsgemäß hergestellten DNN-Isomerengemische enthalten einen überraschend hohen Anteil am 1,5-DNN-Isomer. Üblicherweise liegt der Gehalt an 1,5-DNN über 30 Gew.-%, insbesondere zwischen 34 und 50 Gew.-%. Der Gehalt an anderen Nebenprodukten, insbesondere weiterer Dinitronaphthalinisomeren und höherintrierte Produkten, ist geringer als bei vergleichbaren Nitrierungen mit Mischsäure.

Es kann als Ausgangsprodukt reines 1-Nitronaphthalin oder auch ein Nitronaphthalin-Rohgemisch, wie es bei der Nitrierung von Naphthalin als Rohprodukt erhalten wird, eingesetzt werden.

Der fest perfluorierte stark saure Ionenaustauscher katalysiert die Umsetzung zu 1,5-Dinitronaphthalin angereichertem Endprodukt. Derartige perfluorierte stark saure Ionenaustauscher sind im Stand der Technik bekannt und z.B. unter dein Handelsnamen Nafion® kommerziell erhältlich. Bevorzugt wird ein Ionenaustauscherharz unter der Bezeichnung Nafion® NR50 Superacid Catalyst (Fa. Dupont) eingesetzt.

Der als Katalysator eingesetzte Ionenaustauscher ist chemisch inert und kann leicht aus dem Reaktionsansatz, z.B. durch Filtration, entfernt werden. Eine Aufarbeitung wie bei der bei Nitrierungen mit Mischsäure (Salpetersäure/Schwefelsäure-Gemisch) anfallenden verdünnten und organisch kontaminierten Schwefelsäure entfällt beim erfindungsgemäßen Verfahren in vorteilhafter Weise.

Die Konzentration der Salpetersäure liegt zwischen 50 % und 100 %, bevorzugt zwischen 60 % und 80 %. Die Menge an zugegebener Salpetersäure liegt zwischen 1 Äquivalent und 20 Äquivalenten bezogen auf die eingesetzte Menge Nitronaphthalins. Bei einer 65 %igen Salpetersäure entspricht dies 33 bis 92 Gew.-%. Bevorzugt werden Salpetersäuremengen zwischen 3 Äquivalenten (bei 65 %iger Salpetersäure: 62,7 Gew.-%) und 12 Äquivalenten, (entsprechend 87,0 Gew.-% 65 %iger Salpetersäure) eingesetzt.

Üblicherweise wird das Verfahren bei Temperaturen zwischen 20°C und 100°C, vorzugsweise 80°C, durchgeführt.

Um die vollständige Reaktion sicherzustellen wird die Umsetzung unter guter Durchmischung der Reaktion z.B. durch intensives Rühren für eine Reaktionsdauer zwischen 20 Min. und 8 Stunden, z.B. 3 Stunden, ausgeführt.

Das erfindungsgemäße Verfahren kann ohne weiteres Lösungsmittel nur in einem Überschuß an Salpetersäure oder aber gegebenenfalls auch nach Zusatz eines Lösungsmittels durchgeführt werden.

Die 1,5-Selektivität kann dabei überraschenderweise in Gegenwart eines inerten organischen Lösungsmittels noch weiter erhöht werden.

Bevorzugt wird daher das erfindungsgemäße Verfahren in Gegenwart eines inerten organischen Lösungsmittels durchgeführt. Als derartige Lösungsmittel kommen polare, im Reaktionsmedium inerte Verbindungen in Betracht. Beispiele für solche Lösungsmittel sind z.B. Sulfolan oder Nitroalkane wie beispielsweise Nitromethan.

Bevorzugt wird das erfindungsgemäße Verfahren in Gegenwart von Sulfolan als organischem Lösungsmittel durchgeführt.

Nicht umgesetztes Nitronaphthalin, überschüssige Salpetersäure und Lösungsmittel kann in den Prozeß zurückgeführt werden.

Die Menge an zugesetztem organischem Lösungsmittel liegt zwischen 0,1 und 20 Teilen bezogen auf die eingesetzte Menge Nitronaphthalins bevorzugt zwischen 0,5 und 3 Teilen. Die Konzentration der Salpetersäure wird der Menge an zugesetztem Lösungsmittel angepaßt und liegt zwischen 50 % und 100 %, bevorzugt 60-80 %.

Das Dinitronaphthalin-Isomerengemisch kann in bekannter Weise, z.B. durch fraktionierte Kristallisation in die isomeren Dinitronaphthaline getrennt werden. Derartige Isomerentrennungen, z.B. mit Dimethylformamid oder Dichlorethan als Lösungsmittel, sind bereits beschrieben (vgl. Houben-Weyl; Methoden der organischen Chemie, 1971, Vol. 10, Seite 494).

Ein weiterer Gegenstand der Erfindung sind 1,5- und 1,8-Dinitronaphthalin enthaltende Isomerengemische mit einem Gehalt an 1,5-Dinitronaphthalin über 30 Gew.-%, insbesondere zwischen 34 und 50 Gew.-%.

Bevorzugt sind diese Dinitronaphthalin-Isomerengemische nach dem oben beschriebenen erfindungsgemäßen Verfahren erhältlich.

Aus dem erfindungsgemäßen Isomerengemisch kann das 1,5-DNN auf bekannte Weise abgetrennt und zur Herstellung von 1,5-NDA und 1,5-NDI verwendet werden.

Die folgenden Beispiele sollen die Erfindung erläutern, ohne sie jedoch in ihrem Umfang zu begrenzen.

## **Beispiele**

Als Ausgangsverbindung wurde reines 1-Nitronaphthalin verwendet.

Der als Katalysator eingesetzte Ionenaustauscher mit dem Handelsnamen Nafion® NR 50 Superacid Catalyst (Fa. DuPont) wird in den folgenden Beispielen nur kurz Nafion genannt.

### **1. Nitrierung von 1-Nitronaphthalin unter Verwendung von Salpetersäure mit Nafion als Katalysator**

#### 1.1 Nitrierung mit Nafion-H und der 6-fachen molaren Menge an Salpetersäure

Zu 58,2 g 65 % (w/w) -iger Salpetersäure (entspricht 0,6 mol) wurden 10 g Nafion und 17,3 g (0,1 mol) 1-Nitronaphthalin gegeben. Anschließend wurde der Ansatz 3 h bei 80°C gerührt. Die Aufarbeitung erfolgte durch Einbringen des Reaktionsansatzes in 1000 ml Eiswasser, Abfiltrieren und anschließendem Trocknen des Feststoffs. Die Trennung des Dinitronaphthalins vom Katalysator erfolgte durch Extraktion mit Dioxan. Dabei wird der Feststoff in 200 ml Dioxan bei 90°C gegeben, der Katalysator nach 30 min. Rühren abfiltriert und das Dioxan durch Destillation entfernt.

Die Isomerenzusammensetzung des Rückstandes wurde gaschromatographisch bestimmt (HP 5890, Säule: 25 m SE30, Injektor: 300°C, Detektor (FID): 320°C, Temperaturprogramm: Start: 100°C, Heizrate: 10°C/min, Endtemperatur: 320°C, Probenmenge: 2 -%ig in Dioxan, Einspritzmenge: 1 μl).

### 1.2 Nitrierung mit Nafion-H und der 8-fachen molaren Menge an Salpetersäure

Zu 77,6 g 65 % (w/w) -iger Salpetersäure (entspricht 0,8 mol) wurden 10 g Nafion und 17,3 g (0,1 mol) 1-Nitronaphthalin gegeben. Anschließend wurde der Ansatz 3 h bei 80°C gerührt. Die Aufarbeitung und Bestimmung der Isomerenzusammensetzung erfolgte wie unter 1.1.

### 1.3 Nitrierung ohne Nafion-H und der 6-fachen molaren Menge an Salpetersäure (Vergleichsbeispiel)

Zu 58,2 g 65 % (w/w) -iger Salpetersäure (entspricht 0,6 mol) wurden 17,3 g (0,1 mol) 1-Nitronaphthalin gegeben. Nafion wurde nicht zugegeben. Anschließend wurde der Ansatz 3 h bei 80°C gerührt. Die Aufarbeitung erfolgte durch Einbringen des Reaktionsansatzes in 1000 ml Eiswasser und Abfiltrieren des Feststoffs. Die Bestimmung der Isomerenzusammensetzung erfolgte wie unter 1.1.

### 1.4 Nitrierung mit $H_2SO_4$ und der 6-fachen molaren Menge an Salpetersäure (Vergleichsbeispiel)

Zu 58,2 g 65 % (w/w) -iger Salpetersäure (entspricht 0,6 mol) und 10,0 g Schwefelsäure (0,1 mol) wurden 17,3 g (0,1 mol) 1-Nitronaphthalin gegeben. Anschließend wurde der Ansatz 3 h bei 80°C gerührt. Die Aufarbeitung erfolgte durch Einbringen des Reaktionsansatzes in 1000 ml Eiswasser und Abfiltrieren des Feststoffs. Die Bestimmung der Isomerenzusammensetzung erfolgte wie unter 1.1.

In der Tabelle 1 sind die experimentell ermittelten Isomerenzusammensetzungen wiedergegeben. Bei Nitrierungen mit Nafion ergibt sich eine höhere 1,5-Ausbeute und ein verbessertes Verhältnis (1,5)/(1,8)-DNN. Dies entspricht einer entsprechenden Erhöhung der 1,5-Selektivität bei der Nitrierung.

Tabelle 1

| Zusammensetzung des Produktgemisches bei der Nitrierung von 1-Nitronaphthalin mit Salpetersäure (Beispiele 1.1 bis 1.4); angegeben sind die Äquivalente Nitrierungsmittel, die Konzentration der eingesetzten Salpetersäure und die Reaktionstemperatur. Die Zusammensetzung des Produktgemisches (in Prozent) erfolgte gaschromatographisch. |
|---|

Angegeben ist der Anteil an 1-Nitronaphthalin (1-MNN), 1,5-Dinitronaphthalin (1,5-DNN), 1,8-Dinitronaphthalin (1,8-DNN) und die Summe der Anteile anderer DNN-Isomere und Trinitronaphthalin (DNN+TNN), sowie das Verhältnis der gebildeten Produkte 1,5-DNN und 1,8-DNN ($\frac{1,5\text{-DNN}}{1,8\text{-DNN}}$).

| Bsp. | $HNO_3$ | Katalysator | 1-MNN | 1,5-DNN | 1,8-DNN | $\frac{1,5\text{-DNN}}{1,8\text{-DNN}}$ | Σ der anderen DNN & TNN |
|---|---|---|---|---|---|---|---|
| 1.1 | 6x65 %, 80°C | Nafion | 22,1 | 34,1 | 38,0 | 0,90 | 5,8 |
| 1.2 | 8x65 %, 80°C | Nafion | 2,7 | 43,5 | 49,3 | 0,88 | 4,5 |
| 1.3 | 6x65 %, 80°C | - | 33,8 | 22,5 | 36,3 | 0,62 | 7,4 |
| 1.4 | 6x65 %, 80°C | $H_2SO_4$ | 0,1 | 29,6 | 57,3 | 0,52 | 13,0 |

**2. Nitrierung von 1-Nitronaphthalin unter Verwendung von Salpetersäure mit Nafion als Katalysator in Gegenwart eines organischen Lösungsmittels**

2.1 Nitrierung mit Nafion-H und 6 mol Salpetersäure in Gegenwart von Sulfolan

Zu 58,2 g 65 % (w/w) -iger Salpetersäure (entspricht 0,6 mol) wurden 10 g Nafion, 30 g Sulfolan und 17,3 g (0,1 mol) 1-Nitronaphthalin gegeben. Anschließend wurde der Ansatz 3 h bei 100°C gerührt. Die Aufarbeitung und Bestimmung der Isomerenzusammensetzung erfolgte wie unter 1.1.

2.2 Nitrierung mit Nafion-H und 6 mol Salpetersäure in Gegenwart von Nitromethan

Zu 58,2 g 65 % (w/w) -iger Salpetersäure (entspricht 0,6 mol) wurden 10 g Nafion, 30 g Nitromethan und 17,3 g (0,1 mol) 1-Nitronaphthalin gegeben. Anschließend wurde der Ansatz 3 h bei 100°C gerührt. Die Aufarbeitung und Bestimmung der Isomerenzusammensetzung erfolgte wie unter 1.1.

In der Tabelle 2 sind die experimentell ermittelten Isomerenzusammensetzungen wiedergegeben. Bei Zusatz des Lösungsmittels ergeben sich höhere Verhältnisse (1,5)/(1,8)-DNN. Dies entspricht einer entsprechenden Erhöhung der 1,5-Selektivität bei der Nitrierung.

Tabelle 2

| Zusammensetzung des Produktgemisches bei der Nitrierung von 1-Nitronaphthalin mit Salpetersäure und Zusatz eines Lösungsmittels (Beispiele 2.1 bis 2.2); angegeben sind die Äquivalente Nitrierungsmittel, die Konzentration der eingesetzten Salpetersäure und die Reaktionstemperatur. Die Zusammensetzung des Produktgemisches (in Prozent) erfolgte gaschromatographisch. | | | | | | | |
|---|---|---|---|---|---|---|---|
| Bsp. | $HNO_3$ | Katalysator/ Lösungsmittel | 1-MNN | 1,5-DNN | 1,8-DNN | $\frac{1,5\text{-DNN}}{1,8\text{-DNN}}$ | Σ der anderen DNN & TNN |
| 2.1 | 6x65 %, 100°C | Nafion/ Sulfolan | 68,9 | 15,0 | 9,1 | 1,65 | 7,0 |
| 2.2 | 8x65 %, 100°C | Nafion/ Nitromethan | 55,6 | 18,7 | 17,9 | 1,04 | 7,8 |

**Patentansprüche**

**1.** Verfahren zur Herstellung eines Dinitronaphthalin-Isomerengemisches durch Nitrierung von 1-Nitronaphthalin, **dadurch gekennzeichnet, daß** man Nitronaphthalin gegebenenfalls in einem inerten organischen Lösungsmittel mit der 1 bis 20fachen molaren Menge, bezogen auf die Menge an Nitronaphthalin, an 50 bis 100 %iger Salpetersäure in Gegenwart eines festen perfluorierten stark sauren Ionenaustauschers umsetzt.

**2.** Verfahren nach Anspruch 1, **dadurch gekennzeichnet, daß** als 1-Nitronaphthalin ein Nitronaphthalin-Rohgemisch eingesetzt wird.

**3.** Verfahren nach Anspruch 1 oder 2, **dadurch gekennzeichnet, daß** man als inertes organisches Lösungsmittel Nitroalkane und/oder Sulfolan einsetzt.

**4.** Verfahren nach Anspruch 3, **dadurch gekennzeichnet, daß** man das inerte organische Lösungsmittel in einer Menge von 0,1 bis 20 Gew.-Teilen, bezogen auf die Menge an Nitronaphthalin, einsetzt.

**Claims**

**1.** Process for the production of a dinitronaphthalene isomer mixture by nitration of 1-nitronaphthalene, **characterised in that** nitronaphthalene, optionally in an inert organic solvent, is reacted with 1 to 20 times the molar amount, referred to the amount of nitronaphthalene, of 50 to 100% nitric acid in the presence of a solid, perfluorinated, strongly acidic ion exchanger.

2. Process according to claim 1, **characterised in that** a crude nitronaphthalene mixture is used as 1-nitronaphthalene.

3. Process according to claim 1 or 2, **characterised in that** nitroalkanes and/or sulfolane is/are used as inert organic solvent.

4. Process according to claim 3, **characterised in that** the inert organic solvent is used in an amount of 0.1 to 20 parts by weight, referred to the amount of nitronaphthalene.

**Revendications**

1. Procédé pour la préparation d'un mélange de dinitronaphtalènes isomères par nitration du 1-nitronaphtalène, **caractérisé en ce que** l'on fait réagir le nitronaphtalène éventuellement dans un solvant organique inerte avec 1 à 20 fois la quantité molaire, par rapport à la quantité de nitronaphtalène, d'acide nitrique à une concentration de 50 à 100 % en présence d'un échangeur d'ions perfluoré solide, fortement acide.

2. Procédé selon la revendication 1, **caractérisé en ce que** le 1-nitronaphtalène mis en oeuvre consiste en un mélange brut de nitronaphtalènes.

3. Procédé selon la revendication 1 ou 2, **caractérisé en ce que** le solvant organique inerte utilisé consiste en un nitroalcane et/ou en sulfolan.

4. Procédé selon la revendication 3, **caractérisé en ce que** le solvant organique inerte est mis en oeuvre en quantité de 0,1 à 20 parties en poids par rapport à la quantité de nitronaphtalène.